(19) Europäisches Patentamt
European Patent Office
Office européen des brevets

(11) EP 2 086 513 B1

(12) EUROPEAN PATENT SPECIFICATION

(45) Date of publication and mention of the grant of the patent:
**07.09.2016 Bulletin 2016/36**

(21) Application number: **07808368.0**

(22) Date of filing: **20.09.2007**

(51) Int Cl.:
***A61K 9/16*** *(2006.01)* ***A61K 9/14*** *(2006.01)*
***A61K 9/00*** *(2006.01)* ***A61K 9/19*** *(2006.01)*

(86) International application number:
**PCT/KR2007/004585**

(87) International publication number:
**WO 2008/038944 (03.04.2008 Gazette 2008/14)**

(54) **SUBMICRON NANOPARTICLE OF POORLY WATER SOLUBLE CAMPTOTHECIN DERIVATIVES AND PROCESS FOR PREPARATION THEREOF**

SUBMIKRON-NANOTEILCHEN VON SCHWER WASSERLÖSLICHEN CAMPTOTHECIN-DERIVATEN UND HERSTELLUNGSVERFAHREN DAFÜR

NANOPARTICULE SUBMICRONIQUE DE DÉRIVÉS DE CAMPTOTHÉCINE FAIBLEMENT SOLUBLES DANS L’EAU ET SON PROCÉDÉ DE PRÉPARATION

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **26.09.2006 KR 20060093832**

(43) Date of publication of application:
**12.08.2009 Bulletin 2009/33**

(73) Proprietor: **Samyang Biopharmaceuticals Corporation**
**Seoul 110-725 (KR)**

(72) Inventors:
- **KANG, Hye Won**
  **Daejeon 305-348 (KR)**
- **YIM, Eun Young**
  **Incheon 406-712 (KR)**
- **SEO Min Hyo**
  **Daejeon 302-775 (KR)**

(74) Representative: **Crump, Julian Richard John et al**
**Beresford Crump LLP**
**16 High Holborn**
**London WC1V 6BX (GB)**

(56) References cited:
**EP-A1- 1 666 031** **WO-A1-01/49268**
**WO-A1-99/39700** **WO-A1-2006/049447**
**WO-A1-2006/050123** **US-A1- 2003 082 234**
**US-A1- 2003 215 492** **US-A1- 2004 009 229**

- **MIURA H ET AL: "Antitumor characteristics of methoxypolyethylene glycol-poly(dl-lactic acid) nanoparticles containing camptothecin", JOURNAL OF CONTROLLED RELEASE, ELSEVIER, AMSTERDAM, NL, vol. 97, no. 1, 31 May 2004 (2004-05-31), pages 101-113, XP004508979, ISSN: 0168-3659, DOI: 10.1016/J.JCONREL.2004.03.009**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

[Technical Field]

The present invention relates to a nanoparticle composition comprising a camptothecin derivative, solid polyethyleneglycol and an anti-associative agent, and the process for preparing the same. Specifically, the present invention provides a composition comprising a nanoparticle of the camptothecin derivative, which is prepared by solid-dispersing the poorly water soluble camptothecin derivative in solid polyethyleneglycol and dissolving the solid dispersions in an aqueous solution containing an anti-associative agent. The composition of the present invention stabilizes the camptothecin derivative lactone form having a physiological activity in an aqueous solution of pH 4 to 7, and so can be used as an anticancer agent or for the treatment of cell division-related diseases.

[Background Art]

7-Ethyl-10-hydroxycamptothecin, known as SN-38, is an active metabolite of the commercially available anticancer agent Irinotecan (CPT-11). SN-38 is reported to induce the cell death through the inhibition of DNA synthesis during the cell division by binding to topoisomerase I, an enzyme that participates in the cell division process.

However, SN-38 has poor water solubility, i.e., of 10 $\mu$g/m$\ell$ or less, and therefore, it is difficult to develop SN-38 as a clinical product. For this reason, SN-38 was converted into a prodrug having higher solubility in water, i.e., CPT-11, which has been commercialized. When CPT-11 is administered to the human body, it is metabolized by the enzyme carboxy esterase in liver or cancer cells to the physiologically active SN-38 which displays anticancer effect. However, it has been reported that the conversion rate of CPT-11 into the active SN-38 in the human body is only about 10% or less.

Compared to CPT-11, SN-38 is about 1,000 times or more better in suppressing the activity of topoisomerase I, and 2,000 times or more better in in vitro cytotoxicity.

Further, it has been known that SN-38 exists as the active lactone form under acidic conditions, and as the inactive carboxy anion form under basic conditions, depending on the pH of the aqueous solution. The carboxy anion form of SN-38 can be dissolved in water in the amounts of 4 mg/m$\ell$ or more, but its active lactone form has water solubility of 10 $\mu$g/m$\ell$ or less.

Thus, if SN-38 can be solubilized in a clinically significant concentration or more, it can be developed as an excellent anticancer agent. For this reason, researches about the composition comprising SN-38 to be administered to the human body were performed.

US 5,447,936, US 5,859,023, US 5,674,874, US 5,958,937, US 5,900,419, etc. disclose compositions obtained by dissolving SN-38 in polar organic solvents, such as dimethylacetamide, N-methyl-2-pyrrolidone, dimethylisosorbide, etc. However, the amounts of such polar organic solvents that can be tolerated in the human body are limited, and because the drug may precipitate when mixed with water, intravenous injection of the drug is restricted. Furthermore, when the composition solubilized in an organic solvent is exposed to in vivo condition of pH 7.4, the lactone form that is essential for the activity of SN-38 immediately decomposes to its carboxy anion form.

Miura, H. et al, 'Antitumor Characteristics of Methoxypolyethylene glycol-poly (d1-lactic acid) nanoparticles containingcamptothecin' Journal of controlled release, vol. 97(1), 2004 discloses nanoparticles comprising camptothecin and block copolymer mPEG-PLA.

US-A-2003/0215492 describes a liposome preparation that is obtained by forming a complex of SN-38 and lipid. In this invention, SN-38 in the carboxy anion form is formed in an aqueous solution of pH 8-10, a liposome preparation is obtained therefrom, and SN-38 in the lactone form is prepared under the acidic condition. WO 2002/58622 describes a liposome composition comprising SN-38 and the process for preparing the same, and US-A-2004/0009229 describes a nanoparticle composition prepared from a complex of camptothecin with stabilizing agents, such as polymer, lipid, etc. In this literature, for providing a composition wherein nanoparticles having a particle size of scores or hundreds of nanometers are stably suspended in an aqueous solution, SN-38 is not heated or pulverized, but combined with a polymer or lipid to form a nanoparticle of SN-38/polymer or SN-38/lipid complex. However, the above literatures do not mention whether the lactone form of camptothecin is stably maintained. Another negative aspect of the composition is that the preparation process is very complicated due to the formation step of a complex with a polymer, lipid, etc.

Further, the camptothecin derivatives, particularly SN-38, have very poor solubility in water, and so their formulation is not easy. When they are solubilized according to conventional solubilization technique, they are easily converted to their inactive form, i.e., carboxy anion form, in body fluid (pH 7.4).

[Disclosure]

[Technical Problem]

The present inventors have confirmed that the lactone form of camptothecin derivatives that is stably maintained in body fluid can be obtained by a process comprising melting the camptothecin derivatives in solid polyethyleneglycol under a high temperature, quick-cooling, and then dissolving it in water to produce the nanoparticle of SN-38, and then completed the present invention.

Particularly, the distinction of the present invention is that the camptothecin derivatives are solid-dispersed in the water soluble polymer polyethyleneglycol which is then dissolved in water to effectively produce the nanoparticle of camptothecin derivatives, instead of pulverizing the camptothecin derivative or forming a complex of the camptothecin derivatives with a polymer, etc.

The object of the present invention is to formulate the camptothecin derivatives into a preparation that can be clinically applied to the human body. The inventors have confirmed that the camptothecin derivatives that are converted to the form of a submicron nanoparticle show more excellent lactone stability in the blood than the existing compositions solubilized by polar organic solvent, micelle, etc. when administered to the body via intravenous injection.

[Description of Drawings]

Figure 1 is 1H-NMR spectrum of monomethoxypolyethyleneglycol-polylactide block copolymer (mPEG-PLA) prepared in Preparation 1.

Figure 2 is 1H-NMR spectrum of mPEG-PLA-tocopherol succinate prepared in Preparation 2.

Figure 3 is a graph showing the changes in the median relative tumor volume (RTV) over time using human large intestine cancer cell line HT-29 in a mouse injected with SN-38-containing nanoparticle composition, a comparative preparation or a control.

Figure 4 is a graph showing the changes in the median relative tumor volume (RTV) over time using human pancreas cancer cell line MIA-PaCa-2 in a mouse injected with SN-38-containing nanoparticle composition, a comparative preparation or a control.

[Best Mode]

The present invention relates to a nanoparticle as defined in claim 1.

The present invention further relates to the corresponding process for preparing the nanoparticle composition comprised of the following steps:

(a) melting a camptothecin derivative in solid polyethyleneglycol;
(b) forming a solid dispersion by cooling the melt obtained in step (a); and
(c) dissolving the solid dispersion obtained in step (b) in an aqueous solution of solid surfactant.

The camptothecin derivative-containing nanoparticle composition of this invention and the process for preparing the same are explained in detail below.

In the present invention, the 'nanoparticle' means a small particle in nano size containing a dispersed drug. Its minute size does not cause the blockade of a capillary tube or syringe needle, and therefore, can be administered via intravenous injection. The camptothecin derivative-containing nanoparticle according to the present invention is a composition in the form of a suspension wherein the nanoparticle containing a camptothecin derivative is suspended in an aqueous solution. The size of nanoparticle is preferably in the range of 100 to 1000nm.

The active component of the present invention, the poorly water soluble camptothecin derivative, has a water solubility of 10 $\mu$g/m$\ell$ or less. Thus, it is impossible to clinically use the camptothecin derivative without applying a special solubilization technique. The camptothecin derivative is a compound that substantially has the lactone form of camptothecin. The camptothecin derivative in the present invention is camptothecin or 7-ethyl-10-hydroxycamptothecin (SN-38).

The solid polyethyleneglycol of the present invention is used as a medium for dispersing the camptothecin derivative. It exists as a solid at room temperature, and may have a melting point of 40~60°C. The weight average molecular weight of polyethyleneglycol is between 1,500 to 20,000 Dalton, but more preferably between 2,000 to 10,000 Dalton, and most preferably between 2,000 to 6,000 Dalton. Polyethyleneglycol can exist as a solid at room temperature when its weight average molecular weight is 1,500 Dalton or more. The problem of high viscosity results when its weight average molecular weight exceeds 20,000 Dalton. Polyethyleneglycol can exist in the linear or branched structure, among which the linear one is preferred. Both ends of polyethyleneglycol are preferably protected by hydroxy, alkyl, preferably $(C_{1-4})$alkyl, or acyl, preferably alkylcarbonyl or arylcarbonyl (the aryl includes phenyl, naphthyl, etc.), for example, $(C_{1-18})$acyl, but more preferably by hydroxy. Particularly in carrying out the present invention, the preferable polyethyleneglycol has a pH value of 4 to 8 in an aqueous solution. Polyethyleneglycol having a pH value exceeding 8.0 may play a role in changing the lactone of SN-38 to the carboxy form and therefore, not desirable.

The nanoparticle size increases as the content of the drug camptothecin derivative with respect to polyethyleneglycol increases. Thus, it is preferable to use polyethyleneglycol in amounts of 50 to 1000 folds with respect to the weight of the camptothecin derivative in order to maintain the nanoparticle size at 100 to 1000nm.

The nanoparticle of the present invention includes an anti-associative agent as an essential component. If the solid dispersion wherein the camptothecin derivative is dispersed in solid polyethyleneglycol is suspended in an aqueous solution and left as it is, association between the nanoparticles may occur resulting in the increase of the particle size. To prevent such association between the nanoparticles, the anti-associative agent must be used in the present invention.

As the anti-associative agent, a solid surfactant is preferred. Because the nanoparticle composition is preferably freeze-dried in the final stage to keep the composition stable during long-term storage, the anti-associative agent must exist as a solid at room temperature. Therefore, the solid amphiphilic block copolymer or the solid surfactant should be a solid at room temperature. The anti-associative agent preferably has a melting point of 30°C or more and a water solubility of 1 mg/mℓ or more, should form a micelle in an aqueous solution, and should not show any toxicity, such as producing an oversensitive reaction, etc. when applied to the human body.

The solid surfactant is preferably polyethyleneglycol tocopherol succinate (TPGS). The weight average molecular weight of said polyethyleneglycol is in the range of 1,000 to 5,000 Dalton. The surfactant can exist as a solid at room temperature only when the weight average molecular weight of the polyethyleneglycol is 1,000 Dalton or more. If the weight average molecular weight exceeds 5,000 Dalton, the problem of high viscosity may result, which is not desirable. Thus, such physiologically acceptable and typically used surfactants as polysorbate, chremophore, etc. are not appropriate as the anti-associative agent for the nanoparticle in the present invention, since they all exist as a liquid at room temperature.

The content of the solid surfactant is preferably in the range of 1 to 400 times the weight of the camptothecin derivative, but more preferably 10 to 200 times.

The nanoparticle of the present invention may be formulated into various pharmaceutical forms for administration purpose. To prepare an anticancer composition, the nanoparticle of the present invention is thoroughly mixed with a pharmaceutically acceptable carrier. These pharmaceutical compositions are preferably formulated to a unit dosage form that can be orally, parenterally, subcutaneously, rectally or topically administered for their systemic or topical effect. Any usual pharmaceutical carriers such as water, glycols, oils, alcohols and the like may be acceptable for oral liquid preparations, and such excipients as starches, sugars, kaolin, lubricants, binders, disintegrating agents and the like may be acceptable for oral solid preparations. For injectable preparation, the carriers may include other components such as semipolar solvent as a dissolution aid, but should include considerable amounts of sterile water. As the carriers in the injectable solutions, saline, glucose solution or a mixture of saline and glucose may be used. Injectable suspensions can be prepared using suitable liquid carriers, suspending agents, etc.

The process for preparing the nanoparticle composition according to the present invention comprises the following steps:

(a) melting a camptothecin derivative in solid polyethyleneglycol;
(b) forming a solid dispersion by cooling the melt obtained in step (a); and
(c) dissolving the solid dispersion obtained in step (b) in an aqueous solution of solid surfactant.

In step (a), the poorly water soluble camptothecin derivative is mixed with the solid polyethyleneglycol, and heated preferably to the temperature of 60~180°C. While heating, the mixture should be constantly stirred to melt the camptothecin derivative in the solid polyethyleneglycol. The melting temperature must be 60°C or higher for the solid polyethyleneglycol to melt, and continuous stirring aids the poorly water soluble camptothecin derivative to dissolve in the polyethyleneglycol. The melting temperature must be 180°C or less in order to prevent the decomposition of the camptothecin derivative and polyethyleneglycol and to melt them stably. Considering the solubility of the camptothecin derivative in the polyethyleneglycol, the camptothecin derivative should be mixed with the solid polyethyleneglycol in the ratio that can be thoroughly melted. Preferably, the solid polyethyleneglycol is mixed with the camptothecin derivative in the weight ratio of 50 to 1000 times the weight of the camptothecin derivative.

On the other hand, an organic solvent having a low boiling point selected from methanol, ethanol, dichloromethane, acetone and acetonitrile may be optionally used in the above melting step to effectively melt the poorly water soluble camptothecin derivative in the solid polyethyleneglycol. The melt solution of camptothecin derivative and solid polyethyleneglycol is prepared by adding a suitable organic solvent, which is then removed under reduced pressure. If the solution is continuously heated to the temperature of 60~180°C and stirred even after the organic solvent is removed, the state wherein the camptothecin derivative is melted in polyethyleneglycol can be satisfactorily maintained.

In step (b), the melt solution obtained from step (a) wherein the camptothecin derivative is melted in polyethyleneglycol is quickly cooled preferably to the temperature of 0°C or less to form a solid dispersion. It is preferable that the melt solution is cooled as rapidly as possible using, for example, liquid nitrogen. If the melt solution is slowly cooled, polyethyleneglycol may crystallize, resulting in increase of the particle size of the poorly water soluble camptothecin derivative.

In step (c), the solid dispersion obtained from step (b) is dissolved in an aqueous solution containing an anti-associative agent selected from amphiphilic block copolymers and solid surfactants to give an aqueous suspension of the camptothecin derivative-containing nanoparticle. Polyethyleneglycol can be dissolved in an aqueous solution, but the camptothecin derivative dispersed in polyethyleneglycol does not dissolve in water. Thus, polyethyleneglycol is dissolved, and the camptothecin derivative dispersed in the size of nanoparticle is suspended in the aqueous solution. If the aqueous solution shows an alkali of pH 7.5 or more, the poorly water soluble camptothecin derivative is converted to its carboxy anion form, and therefore, it is preferable to maintain the aqueous solution of the nanoparticle at pH 4~7 to prevent such conversion. The pH regulator that can be used is preferably citric acid, acetic acid, tartaric acid, carbonic acid, lactic acid, sulfuric acid, phosphoric acid, or their alkali salts, such as, sodium salt or potassium salt, or ammonium salt, and most preferably lactic acid.

It is possible that the camptothecin derivative is contained in the above prepared nanoparticle suspension in a concentration range of 0.1~4 mg/mℓ, but preferably in a range of 0.2~2 mg/mℓ.

The solid dispersion, wherein the camptothecin derivative is dispersed in solid polyethyleneglycol, is dissolved in an aqueous solution containing the anti-associative agent to prepare an aqueous solution in which the camptothecin derivative-containing nanoparticle is suspended. Here, it is preferable that the anti-associative agent is dissolved in said aqueous solution in a concentration of 1~200 mg/mℓ, and the pH range of the solution is 4.0~7.0. In order to increase the dissolution rate of the solid polyethyleneglycol in water, it is possible to raise the temperature to 0 to 60°C.

Preferably, the dissolution is performed at a temperature of 30°C or less. Sonication may be used to increase the dissolution rate of polyethyleneglycol in water, and to induce a homogeneous dispersion of the poorly water soluble camptothecin derivative-containing nanoparticle.

The process for preparing the nanoparticle according to the present invention may comprise an additional step (d) for freeze-drying the aqueous solution obtained from step (c).

In step (d), the camptothecin derivative-containing nanoparticle suspended in an aqueous solution, obtained from step (c), is freeze-dried, during which a freeze-drying aid selected from lactose, mannitol and sorbitol is preferably added to the aqueous solution.

The nanoparticle of the present invention prepared as above in the freeze-dried form may be used by diluting or reconstructing it in injectable water or saline. If the nanoparticle of the present invention is reconstructed with injectable water, saline, 5% dextrose solution, etc. into the concentration of camptothecin derivative of 0.1~1.0 mg/mℓ, the amount of active lactone form of camptothecin derivative in the solution is substantially 100%. This solution may be orally or parenterally administered.

**Comparative Preparation 1**

**Polymerization of monomethoxypolyethyleneglycol-polylactide (mPEG-PLA) block copolymer (AB type)**

500g of monomethoxy polyethyleneglycol (weight average molecular weight; Mw: 2,000) was introduced to 100ml 2-neck round-bottomed flask, and heated to 100°C for 2~3 h under reduced pressure to remove water. The reaction flask was filled with dry nitrogen gas. The reaction catalyst stannous octoate [Sn(Oct)2] was added using a syringe in an amount of 0.1% by weight (g, 2.5mol) with respect to D,L-lactide. After stirring for 30 min the mixture was subjected to the conditions of 130°C and reduced pressure (1mmHg) for 1 h to remove the solvent (toluene) in which the catalyst was dissolved. 1375g of the purified lactide was added, and heated for 18 h at 130°C. The resulting polymer was dissolved in methylene chloride, and precipitated by the addition of diethylether. The polymer thus obtained was dried for 48 h in a vacuum oven. The mPEG-PLA obtained had the weight average molecular weight of 1,765~2,000 Dalton, and was confirmed by 1H-NMR to be the A-B type (Figure 1).

**Preparation 2**

**Synthesis of mPEG-PLA-tocopherol succinate**

The mPEG-PLA (10g) obtained in Preparation 1 and tocopherol succinate (Sigma Co., 1.55g; 1.2 times the molar amount of the polymer) were reacted with dicyclohexylcarbodiimide (DCC; 0.76g) and the catalyst dimethylaminopyridine (DMAP; 0.045g) in the solvent acetonitrile (50ml) for 24 h at room temperature. After completion of the reaction, the side product dicyclohexylcarbourea (DCU) was removed by filtration through a glass filter. The residual catalyst was removed by extraction with aqueous hydrochloric acid solution. To the purified solution of the product, magnesium sulfate was added to remove the residual moisture. The product was precipitated in a cosolvent system of n-hexane/diethylether (v/v=7/3), and recrystallized to give mPEG-PLA-tocopherol succinate. The precipitated polymer product was filtered and dried under vacuum to produce a white particle (10g; Yield 88.6%) whose identity was confirmed by 1H-NMR (Figure 2).

**Preparation 3**

**Synthesis of mPEG-PLA-palmitate**

The mPEG-PLA (10g) obtained in Preparation 1 and palmitoyl chloride were dissolved in acetonitrile (50ml) and refluxed for 6 h. After completion of the reaction, the reaction product was added to a cosolvent system of n-hexane/diethylether (v/v=7/3) to precipitate mPEG-PLA-palmitate. The polymer precipitate obtained was filtered and dried under vacuum to produce a white solid (12g; Yield 95%).

**[Examples]**

**Comparative Example 1**

**Preparation of SN-38/PEG 4000/mPEG-PLA tocopherol succinate block copolymer nanoparticle**

SN-38 (5mg) and polyethyleneglycol (molecular weight 4000 Dalton, 1000mg) were introduced to a 250mℓ round-bottomed flask which was placed in a 160°C oil bath. While stirring with a magnetic stirrer, the mixture was allowed to stand for 2 h at room temperature to melt SN-38 in polyethyleneglycol. Then, the reaction vessel was cooled to room temperature, and then, drastically cooled by placing the vessel in liquid nitrogen to produce solid polyethyleneglycol in which SN-38 was dispersed. 10mℓ of the aqueous solution wherein the amphiphilic di-block copolymer mPEG-PLA-tocopherol succinate obtained in Preparation 2 was dissolved in the concentration of 50mg/mℓ was added thereto. The solid polyethyleneglycol was dissolved under sonication to give an aqueous solution wherein the SN-38 nanoparticle was suspended. 500mg of lactose monohydrate was added and dissolved. The pH of the aqueous solution was adjusted to 4.0-7.0. The resulting SN-38 nanoparticle- containing aqueous suspension was filtered through a filter having a pore size of 800nm, and freeze-dried. The freeze-dried composition was reconstructed by injectable water, and then the yield of SN-38, the concentration of SN-38 in the aqueous nanoparticle solution after reconstruction, the content of SN-38 lactone and the size of nanoparticle were analyzed.

The yield of sample was determined from the content of SN-38 in the finally recovered SN-38 aqueous solution with respect to the initial content of SN-38 through dialysis or centrifugation (30,000xg, 1h) in an appropriate aqueous solvent. The sample was dissolved in methanol, and the concentration of SN-38 in the aqueous sample solution was measured by HPLC. The SN-38 lactone content was determined from the carboxy anion peak at about 4 min and the lactone peak at about 12 min in the HPLC analysis using C18 Vydac column, with calculating the lactone content in the SN-38 aqueous solution with respect to the total carboxy ion content in the basic aqueous solutuion of pH 10 or the total lactone content in the acidic aqueous solution of pH 2.

The particle size of sample was measured by DLS (dynamic light scattering) method.

- PEG 4000/mPEG-PLA-tocopherol succinate block copolymer weight ratio: 2/1
- Yield of SN-38: 98%
- Concentration of SN-38 in the aqueous nanoparticle solution after reconstruction: 0.5 mg/mℓ
- Content of SN-38 lactone: 100%
- Size of nanoparticle: 400 nm

**Comparative Example 2**

**Preparation of SN-38/PEG4000/mPEG-PLA-tocophenrol succinate block copolymer nanoparticle**

SN-38 (10mg) and polyethyleneglycol (molecular weight 4000 Dalton, 3000mg) were introduced to a 500mℓ round-bottomed flask. 100mℓ of methanol and 250mℓ of dichloromethane were added to thoroughly dissolve SN-38 and polyethyleneglycol. The organic solvent was removed under reduced pressure. The resulting mixture was then heated to 160°C, and allowed to stand for 2 h with continuous stirring using a magnetic stirrer to melt SN-38 in polyethyleneglycol.

The reaction vessel was cooled to room temperature, and drastically cooled by placing the vessel in liquid nitrogen to produce solid polyethyleneglycol in which SN-38 was dispersed. 10mℓ of the aqueous solution wherein the amphiphilic di-block copolymer mPEG-PLA-tocopherol succinate obtained in Preparation 2 was dissolved in the concentration of 50mg/mℓ was added thereto. The solid polyethyleneglycol was dissolved under sonication to produce an aqueous solution wherein the SN-38 nanoparticle was suspended. 600mg of lactose monohydrate was added and dissolved. The pH of the aqueous solution was adjusted to 4.0~7.0. The resulting SN-38 nanoparticle-containing aqueous suspension was filtered through a filter having a pore size of 800nm, and freeze-dried. The freeze-dried composition was reconstructed by injectable water, and then the yield of SN-38, the concentration of SN-38 in the aqueous nanoparticle solution after reconstruction, the content of SN-38 lactone and the size of nanoparticle were analyzed.

- PEG 4000/mPEG-PLA-tocopherol succinate block copolymer weight ratio: 6/1
- Yield of SN-38: 99%
- Concentration of SN-38 in the aqueous nanoparticle solution after reconstruction: 0.5 mg/mℓ
- Content of SN-38 lactone: 100%
- Size of nanoparticle: 500 nm

**Comparative Example 3**

**Preparation of nanoparticle composition based on SN-38/PEG4000/mPEGPLA block copolymer**

SN-38 nanoparticle composition was prepared according to the same procedure as Example 1 except that 1g of mPEG-PLA block copolymer (Mw; 1,800-2,000) obtained in Preparation 1, 8g of PEG4000 and 40mg of SN-38 were used. The prepared composition was reconstructed by injectable water, and then the yield of SN-38, the concentration of SN-38 in the aqueous nanoparticle solution after reconstruction, and the content of SN-38 lactone were analyzed.

- PEG 4000/mPEG-PLA block copolymer weight ratio: 8/1
- Yield of SN-38: 90%
- Concentration of SN-38 in the aqueous nanoparticle solution after reconstruction: 0.4 mg/mℓ
- Content of SN-38 lactone: 100%

**Comparative Example 4**

**Preparation of nanoparticle composition based on SN-38/PEG4000/mPEG-PLA- palmitate**

SN-38 aqueous nanoparticle solution was prepared according to the same procedure as Example 1 except that 100mg of mPEG-PLA-palmitate obtained in Preparation 3, 400mg of PEG4000 and 1.0mg of SN-38 were used. The prepared composition was reconstructed by injectable water, and then the yield of SN-38, the concentration of SN-38 in the aqueous nanoparticle solution after reconstruction, and the content of SN-38 lactone were analyzed.

- PEG 4000/mPEG-PLA-palmitate weight ratio: 4/1
- Yield of SN-38: 95%
- Concentration of SN-38 in the aqueous nanoparticle solution after reconstruction: 0.5 mg/mℓ
- Content of SN-38 lactone: 100%

**Example 5**

**Preparation of nanoparticle composition based on SN-38/PEG4000/tocopherol polyethyleneglycol succinate (TPGS)**

SN-38 aqueous nanoparticle solutuion was prepared according to the same procedure as Example 1 except that 70mg of tocopherol polyethyleneglycol succinate (TPGS, vitamin-E polyethyleneglycol-1000-succinate, Eastman Chemical Co., Kingsport Tenn., Mn; 1,000), 21g of PEG4000 and 70mg of SN-38 were used. The prepared composition was reconstructed by injectable water, and then the yield of SN-38, the concentration of SN-38 in the aqueous nanoparticle solution after reconstruction, and the content of SN-38 lactone were analyzed.

- PEG 4000/tocopherol-polyethyleneglycol succinate (TPGS, Mn; 1,000) weight ratio: 300/1
- Yield of SN-38: 93%
- Concentration of SN-38 in the aqueous nanoparticle solution after reconstruction: 0.7 mg/mℓ

- Content of SN-38 lactone: 100%

**Comparative Example 6**

**Preparation of nanoparticle composition based on SN-38-containing solid polyethyleneglycol**

Depending on the molecular weight of the solid polyethyleneglycol, compositions using only the drug and the dispersion medium solid polyethyleneglycol were prepared in the weight ratios shown in the following table. Each composition was prepared according to the same procedure as Example 1. However, in the final step, an aqueous solutuion (1mℓ) of pH 5-6 having no anti-associative agent was added to give the final composition. The nanoparticle size of the obtained composition was determined immediately after the preparation (0 h), and after 4 h and 24 h at room temperature. The composition was filtered through a filter having a pore size of 800nm, and the drug concentration in the filtrate was measured to determine the drug content as shown in Table 1.

[Table 1]

| Component's ratio, particle size and drug content of Compositions 1~9 | | | | | | | | |
|---|---|---|---|---|---|---|---|---|
| | Drug | Dispersion Medium [Solid polyethyleneglycol] | | | Particle Size (nm) | | | Content (mg/mℓ) |
| | SN-38 (mg) | PEG 2000 (mg) | PEG 4000 (mg) | PEG 10,000 (mg) | 0 h | 4 h | 24 h | |
| Composition 1 | 1 | 200 | - | - | 950 | 1300 | 2000 | 0.15 |
| Composition 2 | 1 | 300 | - | - | 930 | 1000 | 1700 | 0.20 |
| Composition 3 | 1 | 400 | - | - | 750 | 900 | 1500 | 0.45 |
| Composition 4 | 1 | - | 200 | - | 600 | 1000 | 1500 | 0.30 |
| Composition 5 | 1 | - | 300 | - | 500 | 900 | 1100 | 0.50 |
| Composition 6 | 1 | - | 400 | - | 400 | 850 | 1000 | 0.65 |
| Composition 7 | 1 | - | - | 200 | 400 | 800 | 1100 | 0.40 |
| Composition 8 | 1 | - | - | 300 | 350 | 750 | 1000 | 0.65 |
| Composition 9 | 1 | - | - | 400 | 400 | 700 | 1000 | 0.75 |

The SN-38 nanoparticle aqueous suspensions prepared using solid polyethyleneglycol without an anti-associative agent showed unstable particle size over time, and resulted in a particle size of 1000nm or more after they were allowed to stand for 24 h at room temperature. Therefore, use of such anti-associative agent as a surfactant is needed for securing the composition's stability.

**Example 7**

**SN-38-containing nanoparticle composition**

To supplement the results of Example 6, anti-associative agents acting as a surfactant were used to prepare nanoparticle compositions containing SN-38. The SN-38 nanoparticle compositions were prepared according to the same procedure as in Example 1 in the weight ratios shown in the following Table 2. To these compostions, D,L-mannitol that is used as a cake substance at the time of freeze-drying was added in the weight ratio of 15% with respect to the total composition. The mixture was stirred for 15 min at room temperature. The obtained aqueous nanoparticle solution was filtered through a filter having a pore size of 800nm, and the equal amounts thereof were distributed to glass vials to include the same content of the drug, and freeze-dried. The freeze-dried compositions were reconstructed with saline to the concentration of 0.5mg/mℓ. The particle size and pH of SN-38 at 0 h (immediately after reconstruction), 4 h and 24 h at room temperature were measured and shown in Table 2.

[Table 2]

| Component's ratio, particle size and drug content of Compositions 10~20 | | | | | | | |
|---|---|---|---|---|---|---|---|
| | Drug (1mg) | Dispersion Medium (300mg) | Anti-associative agent (50mg) | Particle Size (nm) | | | pH |
| | | | | 0 h | 4 h | 24 h | |
| Composition 10 | SN-38 | PEG 2000 | mPEG-PLA-OH [a)] | 930 | 950 | 950 | 4.8 |
| Composition 11 | SN-38 | PEG 4000 | mPEG-PLA-OH [a)] | 805 | 800 | 810 | 5.0 |
| Composition 12 | SN-38 | PEG 10,000 | mPEG-PLA-OH [a)] | 780 | 790 | 807 | 5.3 |
| Composition 13 | SN-38 | PEG 2000 | mPEG-PLA-tocopherol succinate [b)] | 840 | 850 | 880 | 4.9 |
| Composition 14 | SN-38 | PEG 4000 | mPEG-PLA-tocopherol succinate [b)] | 500 | 510 | 550 | 5.3 |
| Composition 15 | SN-38 | PEG 10,000 | mPEG-PLA-tocopherol succinate [b)] | 530 | 530 | 540 | 5.6 |
| Composition 16 | SN-38 | PEG 2000 | TPGS (1mg) [c)] | 513 | 530 | 545 | 5.2 |
| Composition 17 | SN-38 | PEG 4000 | TPGS (1mg) [c)] | 350 | 360 | 365 | 5.6 |
| Composition 18 | SN-38 | PEG 10,000 | TPGS (1mg) [c)] | 300 | 310 | 340 | 6.4 |
| Composition 19 | CPT | PEG 4000 | mPEG-PLA-OH [a)] | 730 | 740 | 740 | 4.9 |
| Composition 20 | CPT | PEG 4000 | mPEG-PLA-tocopherol succinate [b)] | 580 | 590 | 590 | 5.0 |
| * a) Molecular weight of mPEG-PLA-OH: (Dalton) [2K-1800]<br>b) Molecular weight of mPEG-PLA-tocopherol succinate: (Dalton) [2K-860-530]<br>c) TPGS [d-alpha-tocopheryl polyethyleneglycol 1000 succinate] | | | | | | | |

The composition of Table 2 maintained 100% lactone form in an aqueous solutuion for 24 h. The particle size of the drug was stable over time as a result of adding the anti-associative agent.

**[Experiments]**

**Experiment 1**

**Stability of SN-38-containing nanoparticle composition**

Each of the freeze-dried compositions for injection prepared in Examples 2, 3 and 5 was stored for 6 month at 25°C. The stability of the composition was examined by measuring the appearance, retain rate (content), % lactone, time for redissolution, pH change and average nanoparticle size. The results are shown in Table 3.

[Table 3]

| Appearance, retain rate (content), % lactone, time for redissolution, pH change and average nanoparticle size of the freeze-dried compositions for injection (after storage for 6 month at 25°C) | | | | | | |
|---|---|---|---|---|---|---|
| Preparation | Appearance | Content (%) | % Lactone | Time for Redissolution | pH | Average Nanoparticle Size |
| Example 2 | Suitable | 100 | 100 | Within 30 sec | 5.0 | 600 |
| Example 3 | Suitable | 96.7 | 100 | Within 30 sec | 4.5 | 790 |
| Example 5 | Suitable | 98 | 100 | Within 30 sec | 5.2 | 450 |

The freeze-dried compositions prepared according to the present invention remained stable even after long-term storage, and were estimated to be appropriate for clinical application.

**Experiment 2**

**Pharmacokinetic properties of SN-38-containing nanoparticle compositions**

To identify the pharmacokinetic properties of each of the freeze-dried compsotions prepared in Examples 2, 3 and 5, tubings were introduced to the jugular vein and the carotid of male Sprague-Dawley rats weighing in the range of 200~250g. Each composition was injected via a vein in the tail over 10 second time span in a dosage of 2 mg/kg. After 5 min, 15 min, 30 min, 1 h, 2 h, 4 h and 8 h from the time of the injection, a sample 0.4 mℓ of blood was taken from the carotid, and centrifuged to produce a clear supernant plasma.

Methanol of pH 5.5 adjusted by 10% $ZnSO_4$ and 200mM lactate buffer (pH 3.5) was added thereto to analyze the drug concentration in the plasma. This mixture was vigorously mixed for 30 seconds, and then centrifugated. The supernatant was taken, and transferred to a clear tube. The concentration was measured by HPLC under the following conditions:

Injection volume: 0.085 mℓ

Flow rate: 1 mℓ/min

Detector: FLD

Wavelength: Ex 355nm, Em 515nm

Mobile phase: A solvent mixture of acetonitrile/3% aqueous triethylamine solution = 80/20 by volume ratio adjusted to pH 5.5 by using acetic acid

Column: 4.6 x 250mm (C18, Vydac, USA)

The plasma concentration of SN-38 lactone released from the administered nanoparticles was analyzed, and shown in Table 4.

As a comparative preparation, the water soluble prodrug of SN-38, Campto® injection (20 mg/mℓ, CJ Pharma/Pfizer, USA) was used after dilution with saline.

[Table 4]

| Plasma concentration of SN-38 lactone released from the administered nanoparticles | | | | | | | | |
|---|---|---|---|---|---|---|---|---|
| | Dosage (mg/kg) | Plasma concentration of SN-38 lactone (μg/mℓ) | | | | | | |
| | | 5 min | 15 min | 30 min | 1h | 2h | 4h | 8h |
| Example 2 | 2 | 1.8262 | 0.4317 | 0.1873 | 0.0597 | 0.0073 | 0.0035 | 0.0027 |
| Example 3 | 2 | 4.0810 | 0.4582 | 0.1559 | 0.0368 | 0.0083 | 0.0029 | 0.0013 |
| Example 5 | 2 | 1.5190 | 0.1851 | 0.0745 | 0.0293 | 0.0170 | 0.0060 | 0.0066 |
| Comparative preparation | 6 | 0.2139 | 0.1303 | 0.0534 | 0.0705 | 0.0419 | 0.0179 | 0.003 |

**Experiment 3**

**In vivo anticancer activity of SN-38-containing nanoparticle composition**

The effect of the nanoparticle composition prepared in Example 2 was used to estimate the anticancer activity. As a comparative preparation, Campto® injection (20 mg/mℓ, CJ Pharma/Pfizer, USA) was used after dilution with saline to 1.5 mg/mℓ of the CPT-11 content.

Cells stored in liquid nitrogen were taken, and subjected to in vitro cell culture. The cells were harvested, and washed with sterile phosphate buffered saline (PBS), and the living cells were counted. The cells were resuspended in sterile PBS in a population of about 7 x $10^7$ cells/mℓ. 0.1 mℓ of cell suspension containing 7 x $10^6$ human cancer cells (HT-29, MIA-Paca-2) was subcutaneously injected to the right side of a healthy athymic nude mouse (nu/nu; 20-25g, 8 weeks). After the cancer had grown to a certain size, xenotransplantation was performed three times to form a xenograft of 3-4mm. The xenograft was subcutaneously injected to the right side of a healthy athymic nude mouse (nu/nu; 20-25g, 8 weeks) using a 12 gauge troca needle. After the tumor volume reached 100-300 $mm^3$, the drug was administered. The day on which the drug was adminstered was recorded as 0 day. On 0 day, the mice were divided into five (5) groups, SN-38-containing nanoparticle prepared in Example 2 and the comparative preparation were each administered via a vein in the tail on days 0, 1, 2, 3, 4 and 5, and the tumor volume was measured at different time intervals. The tumor volume was calculated according to the following equation.

$$\text{Tumor Volume (TV)} = 0.5 \times L \times W^2 \text{ (L: major axis, W: minor axis)}$$

$$\text{Relative Tumor Volume (RTV)} = (V_t/V_0) \times 100\% \text{ (}V_t\text{: TV at t day, }V_0\text{: TV at 0 day)}$$

The therapeutic efficacy was determined by considering all aspects of the average tumor proliferation curve, the optimal growth suppression (T/C%) and the specific growth delay (SGD).

On a certain day within four weeks after the last injection, the optimal growth suppression was calculated by multiplying the median RTV value of the treated group to control group by 100% (T/C%).

SGD was calculated over 1 to 2 doubling times as follows:

$$\text{Specific Growth Delay (SGD)} = (T_D \text{ treated group} - T_D \text{ control group}) / T_D \text{ control group}$$

$T_D$: Tumor Doubling Time

The activity level was defined as follows.

| | T/C% | | SGD |
|---|---|---|---|
| (+) | <50 | or | >1.0 |
| + | <50 | and | >1.0 |
| ++ | <40 | and | >1.5 |
| +++ | <25 | and | >2.0 |
| ++++ | <10 | and | >3.0 |

In order to recognize the validity of the experiments, at least 7 mice were used per treatment and at least 7 tumors per group were used. At the starting point of the treatment, the initial tumor had a diameter of 4 mm or the volume of 30 $mm^3$. The animals that died within 2 weeks after the final administration of the drug were categorized as toxified death, and excluded from the estimation. The groups having more than one death out of three animals, attributable to toxified death, or the groups having animals that had an average body weight decrease of more than 15% and did not show signs of thorough recovery, were considered to have no anti-tumor activity.

As can be seen from Table 5, Figures 3 and 4, the group treated with the SN-38-containing nanoparticle composition prepared in Example 2 showed considerable inhibitory activity against the growth of the cancer compared with the control group, and particularly higher anticancer activity than that of the comparative preparation.

[Table 5]

<table>
<tr><td colspan="10">Estimation of anticancer activity</td></tr>
<tr><td rowspan="2">Cancer Cell Line</td><td rowspan="2">Administration Schedule (day)</td><td rowspan="2">n</td><td colspan="2">T/C%</td><td colspan="2">SGD</td><td colspan="2">Activity Level</td></tr>
<tr><td>Ex. 2</td><td>Comparative Preparation</td><td>Ex. 2</td><td>Comparative Preparation</td><td>Ex. 2</td><td>Comparative Preparation</td></tr>
<tr><td>HT-29*</td><td>0.1.2.3.4.5 (qld x 5)</td><td>7</td><td>43.10</td><td>56.38</td><td>4.08</td><td>2.05</td><td>+</td><td>(NA)</td></tr>
<tr><td>MIA-Paca-2**</td><td>0.1.2.3.4.5 (qld x 5)</td><td>7</td><td>16.03</td><td>44.05</td><td>11.29</td><td>4.43</td><td>+++</td><td>+</td></tr>
<tr><td colspan="9">* HT-29 (CPT-11 20 mg/kg, SN-38 10 mg/kg)<br>** MIA-PaCa-2 (CPT-11 10 mg/kg, SN-38 10 mg/kg)</td></tr>
</table>

[Industrial Applicability]

The nanoparticle composition, according to the present invention, is a nanoparticle suspension containing the poorly water soluble camptothecin derivative, and is characterized by the delay in the conversion of the active lactone form to the inactive form upon dilution or reconstruction with an aqueous solution such as saline. Also, the present invention is significant in that it provides the solubilization of the poorly water soluble camptothecin derivative, the preparation of nano-size particle, and the development of new drug for treating cancer.

## Claims

1. A nanoparticle composition of camptothecin or 7-ethyl-10-hydroxycamptothecin, which comprises camptothecin or 7-ethyl-10-hydroxycamptothecin, solid polyethyleneglycol, and solid surfactant as an anti-associative agent, wherein the solid polyethyleneglycol has a weight average molecular weight of 1,500 to 20,000 Dalton;
 and
 the solid surfactant is polyethyleneglycol tocopherol succinate whose polyethyleneglycol has a weight average molecular weight of 1,000 to 5,000 Dalton.

2. The composition of Claim 1 wherein both ends of polyethyleneglycol are protected by a hydroxy, alkyl or acyl group.

3. The composition of Claim 1 wherein polyethyleneglycol is contained in an amount of 50 to 1000 folds the weight of the camptothecin or 7-ethyl-10-hydroxycamptothecin.

4. The composition of Claim 1 wherein the anti-associative agent is contained in an amount of 1 to 400 times the weight of the camptothecin or 7-ethyl-10-hydroxycamptothecin.

5. The composition of Claim 1 wherein the size of nanoparticle is in the range of 100 to 1000nm.

6. The composition of Claim 1 wherein the nanoparticle composition is present in the freeze-dried form.

7. The composition of Claim 1, which further comprises a pharmaceutically acceptable carrier, for use in treating cancer.

8. A process for preparing the nanoparticle composition according to Claim 1, which comprises the steps of

 (a) melting camptothecin or 7-ethyl-10-hydroxycamptothecin in solid polyethyleneglycol;
 (b) forming a solid dispersion by cooling the melt obtained in step (a); and
 (c) dissolving the solid dispersion obtained in step (b) in an aqueous solution of solid surfactant,

 wherein the solid polyethyleneglycol has a weight average molecular weight of 1,500 to 20,000 Dalton;
 and
 the solid surfactant is polyethyleneglycol tocopherol succinate whose polyethyleneglycol has a weight average molecular weight of 1,000 to 5,000 Dalton.

**9.** The process of Claim 8, which comprises a further step for freeze-drying the aqueous solution obtained in step (c).

**10.** The process of Claim 8 wherein the melting temperature in step (a) is 60~180°C.

**11.** The process of Claim 8 wherein an organic solvent selected from methanol, ethanol, dichloromethane, acetone and acetonitrile is used in step (a), and the organic solvent is removed under reduced pressure.

**Patentansprüche**

**1.** Nanopartikelzusammensetzung von Camptothecin oder 7-Ethyl-10-hydroxycamptothecin, welche Camptothecin oder 7-Ethyl-10-hydroxycamptothecin, festes Polyethylenglycol und festes Tensid als assoziationsverhinderndes Mittel umfasst,
wobei das feste Polyethylenglycol eine gewichtsmittlere Molmasse von 1.500 bis 20.000 Dalton hat; und
das feste Tensid Polyethylenglycol-Tocopherolsuccinat ist, dessen Polyethylenglycol eine gewichtsmittlere Molmasse von 1.000 bis 5.000 Dalton hat.

**2.** Zusammensetzung nach Anspruch 1, wobei beide Polyethylenglycolenden durch eine Hydroxy-, Alkyl- oder Acylgruppe geschützt sind.

**3.** Zusammensetzung nach Anspruch 1, wobei Polyethylenglycol in einer 50- bis 1000-fachen Menge des Gewichts des Camptothecins oder 7-Ethyl-10-hydroxycamptothecins enthalten ist.

**4.** Zusammensetzung nach Anspruch 1, wobei das assoziationsverhindernde Mittel in einer 1- bis 400-fachen Menge des Gewichts des Camptothecins oder 7-Ethyl-10-hydroxycamptothecins enthalten ist.

**5.** Zusammensetzung nach Anspruch 1, wobei die Nanopartikelgröße im Bereich von 100 bis 1000 nm liegt.

**6.** Zusammensetzung nach Anspruch 1, wobei die Nanopartikelzusammensetzung in gefriergetrockneter Form vorliegt.

**7.** Zusammensetzung nach Anspruch 1, welche weiterhin einen pharmazeutisch verträglichen Träger umfasst, zur Verwendung bei der Behandlung von Krebs.

**8.** Verfahren zur Herstellung der Nanopartikelzusammensetzung nach Anspruch 1, welches die Schritte umfasst:

(a) Schmelzen von Camptothecin oder 7-Ethyl-10-hydroxycamptothecin in festem Polyethylenglycol;
(b) Bilden einer festen Dispersion durch Abkühlen der in Schritt (a) erhaltenen Schmelze; und
(c) Lösen der in Schritt (b) erhaltenen festen Dispersion in einer wässrigen Lösung von festem Tensid,

wobei das festes Polyethylenglycol eine gewichtsmittlere Molmasse von 1.500 bis 20.000 Dalton hat; und
das feste Tensid Polyethylenglycol-Tocopherolsuccinat ist, dessen Polyethylenglycol eine gewichtsmittlere Molmasse von 1.000 bis 5.000 Dalton hat.

**9.** Verfahren nach Anspruch 8, welches einen weiteren Schritt zum Gefriertrocknen der in Schritt (c) erhaltenen wässrigen Lösung umfasst.

**10.** Verfahren nach Anspruch 8, wobei die Schmelztemperatur in Schritt (a) 60~180 °C ist.

**11.** Verfahren nach Anspruch 8, wobei in Schritt (a) ein organisches Lösemittel verwendet wird, das ausgewählt ist aus Methanol, Ethanol, Dichlormethan, Aceton und Acetonitril, und das organische Lösemittel unter reduziertem Druck entfernt wird.

**Revendications**

**1.** Composition de nanoparticules de camptothécine ou de 7-éthyl-10-hydroxycamptothécine, qui comprend de la camptothécine ou de la 7-éthyl-10-hydroxycamptothécine, un polyéthylèneglycol solide, et un tensioactif solide servant d'agent anti-associatif,
dans laquelle le polyéthylèneglycol solide a une masse moléculaire moyenne en masse de 1 500 à 20 000 Daltons ; et le tensioactif solide est un adduit de succinate de tocophérol et de polyéthylèneglycol dont le fragment polyéthylèneglycol a une masse moléculaire moyenne en masse de 1 000 à 5 000 Daltons.

**2.** Composition selon la revendication 1, dans laquelle les deux extrémités du polyéthylèneglycol sont protégées par un groupe hydroxy, alkyle ou acyle.

**3.** Composition selon la revendication 1, dans laquelle le polyéthylèneglycol est présent en une quantité de 50 à 1000 fois le poids de la camptothécine ou de la 7-éthyl-10-hydroxycamptothécine.

**4.** Composition selon la revendication 1, dans laquelle l'agent anti-associatif est présent en une quantité de 1 à 400 fois le poids de la camptothécine ou de la 7-éthyl-10-hydroxycamptothécine.

**5.** Composition selon la revendication 1, dans laquelle la taille des nanoparticules est située dans la plage allant de 100 à 1000 nm.

**6.** Composition selon la revendication 1, dans laquelle la composition de nanoparticules est présente sous forme lyophilisée.

**7.** Composition selon la revendication 1, qui comprend en outre un véhicule pharmaceutiquement acceptable, pour utilisation dans le traitement d'un cancer.

**8.** Procédé pour préparer la composition de nanoparticules selon la revendication 1, qui comprend les étapes de

(a) fusion de camptothécine ou de 7-éthyl-10-hydroxycamptothécine dans du polyéthylèneglycol solide ;
(b) formation d'une dispersion solide par refroidissement de la masse fondue obtenue à l'étape (a) ; et
(c) dissolution de la dispersion solide obtenue à l'étape (b) dans une solution aqueuse de tensioactif solide,

dans lequel le polyéthylèneglycol solide a une masse moléculaire moyenne en masse de 1 500 à 20 000 Daltons ; et le tensioactif solide est un adduit de succinate de tocophérol et de polyéthylèneglycol dont le fragment polyéthylèneglycol a une masse moléculaire moyenne en masse de 1 000 à 5 000 Daltons.

**9.** Procédé selon la revendication 8, qui comprend une étape supplémentaire de lyophilisation de la solution aqueuse obtenue à l'étape (c).

**10.** Procédé selon la revendication 8, dans lequel la température de fusion dans l'étape (a) est de 60 à 180°C.

**11.** Procédé selon la revendication 8, dans lequel un solvant organique choisi parmi le méthanol, l'éthanol, le dichlorométhane, l'acétone et l'acétonitrile est utilisé dans l'étape (a), et le solvant organique est éliminé sous pression réduite.

【Figure 1】

mPEG | PLA

$CH_3O\text{-}(\text{-}CH_2CH_2O\text{-})n\text{-}C\underline{H}_2C\underline{H}_2O\text{-}(CO\text{-}CH(CH_3)\text{-}O\text{-})m\text{-}CO\text{-}C\underline{H}(CH_3)\text{-}OH$

d c c c a e f b f

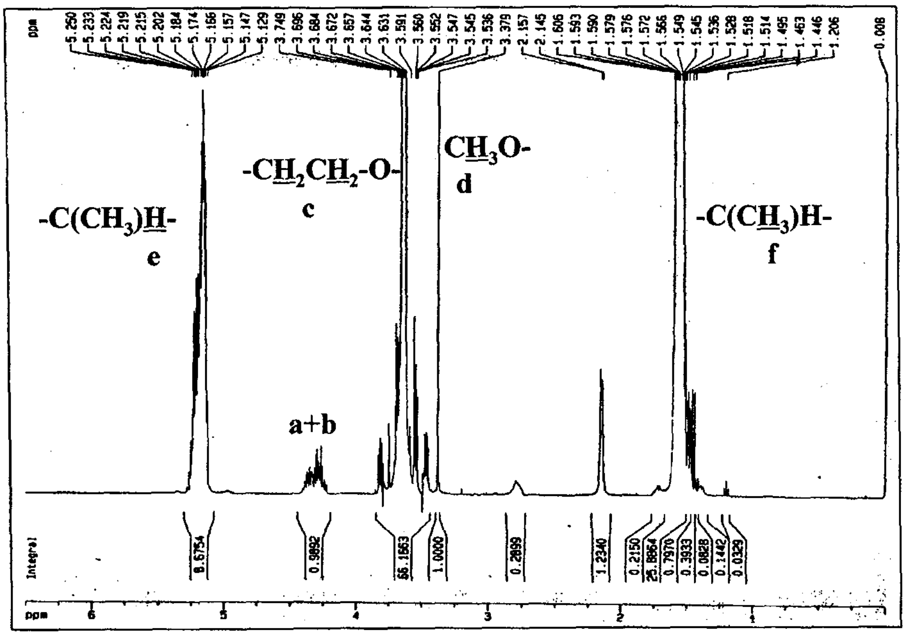

【Figure 2】

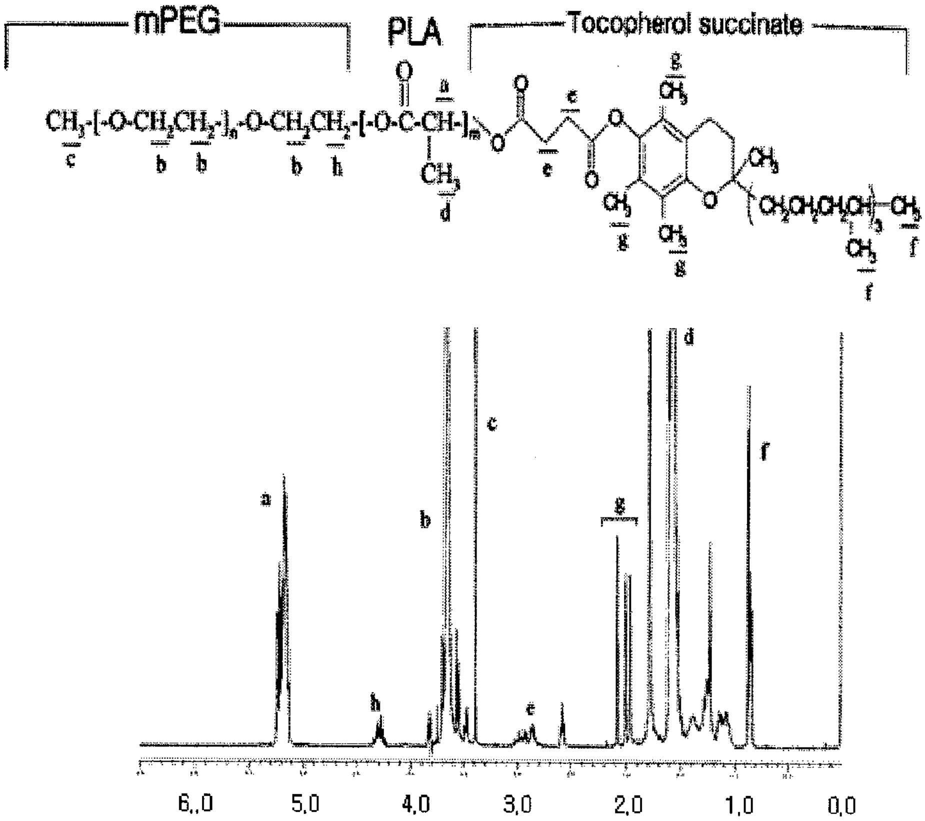

【Figure 3】

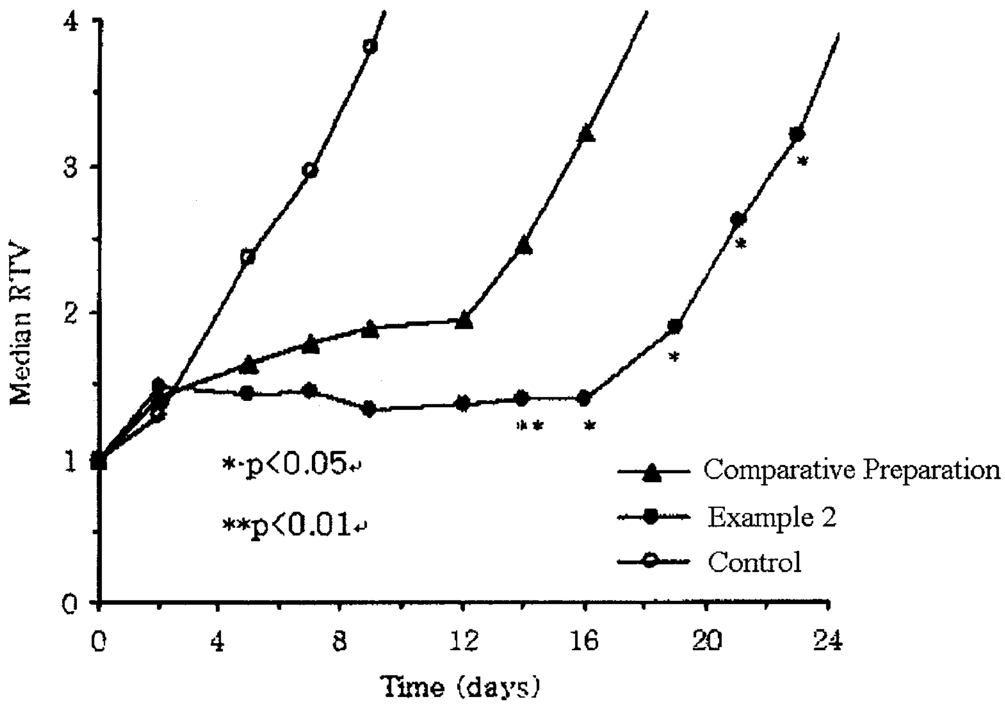
Median RTV
Time (days)
0
1
2
3
4
0
4
8
12
16
20
24
*p<0.05
**p<0.01
**
*
*
*
*
Comparative Preparation
Example 2
Control

【Figure 4】

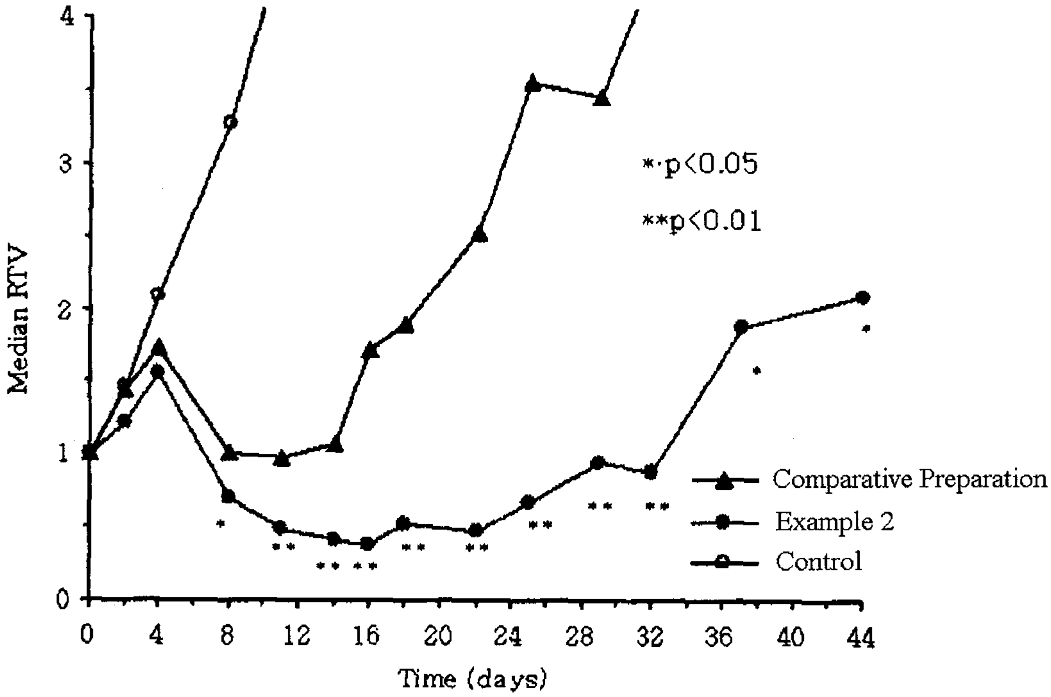

4
3
2
1
0
Median RTV
*·p<0.05
**p<0.01
Comparative Preparation
Example 2
Control
0 4 8 12 16 20 24 28 32 36 40 44
Time (days)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5447936 A
- US 5859023 A
- US 5674874 A
- US 5958937 A
- US 5900419 A
- US 20030215492 A
- WO 200258622 A
- US 20040009229 A


### Non-patent literature cited in the description


- **MIURA, H. et al.** Antitumor Characteristics of Methoxypolyethylene glycol-poly (d1-lactic acid) nanoparticles containingcamptothecin. *Journal of controlled release,* 2004, vol. 97 (1